# EUROPEAN PATENT APPLICATION

(11) **EP 4 830 711 A1**
(43) Date of publication of application: **28.10.2026**
(21) Application number: 25382422.1
(22) Date of filing: 25.04.2025
(51) Int. Cl.: A61K 35/745, A61K 31/4415, A61K 31/51, A61K 31/525, A61K 31/714, A61K 35/74, A61K 35/741, A61K 35/747, A61K 36/06, A61K 36/064, A61P 19/02, A61P 19/10, A61P 21/00

(54) **COMBINATION OF BIOTICS FOR A TREATMENT OF A MEDICAL MUSCULOSKELETAL DEGENERATIVE DISORDER**

(71) Applicant: AB Mauri (UK) Ltd, London W1K 4QY (GB)
(72) Inventor: The designation of the inventor has not yet been filed
(74) Representative: V.O.

(57) **Abstract**

The invention relates to a combination of at least a first bioactive selected from the group consisting of probiotics, parabiotics, postbiotics and prebiotics, and at least a second bioactive selected from the group consisting of probiotics, parabiotics, postbiotics, prebiotics, botanical extracts, omega-3 polyunsaturated fatty acids and further joint-supportive substances, wherein said first bioactive and said second bioactive are different from each other, for use in the treatment of a medical musculoskeletal degenerative disorder. The invention further relates to a pharmaceutical or nutraceutical composition comprising said bioactives.

## Description

The invention relates to a combination of two or more bioactives for use in the treatment of a medical musculoskeletal degenerative disorder. The invention further relates to a pharmaceutical or nutraceutical composition comprising the two or more bioactives.

Musculoskeletal degenerative disorders form a group of medical disorders manifesting itself mainly in the aging people. Osteoporosis, osteopenia, sarcopenia, and osteoarthritis are examples of such medical disorders affecting the musculoskeletal system, which have several aspects in common. They are all age-related medical disorders; they become more prevalent as people age. Age-related hormonal changes, such as decreased estrogen or testosterone levels, can lead to bone and muscle deterioration, contributing to the development of these disorders. Further, they all for adversely affect the musculoskeletal system, leading to symptoms like impaired strength, impaired mobility, or impaired physical function. Furthermore, they are all four associated with an increased risk of fractures and falls. Moreover, they are chronic conditions, often with progressive worsening, if not properly managed. While they do not have immediate life-threatening effects, they can severely reduce the quality of life by increasing pain, disability, and dependence on others. In addition, they share risk factors, such as aging, sedentary lifestyle, malnutrition (notably inadequate intake of calcium, vitamin D, and protein), hormonal changes, metabolic factors and chronic inflammation. In osteoarthritis, inflammation directly contributes to joint damage. For osteoporosis, osteopenia, and sarcopenia, inflammation can accelerate bone and muscle loss. Examples of metabolic factors associated with these disorders are insulin resistance, obesity, and chronic diseases (e.g., diabetes).

Presently known strategies for treatment (which may be a preventive treatment) include exercise: resistance training, weight-bearing exercises, and flexibility exercises are beneficial for all four conditions. Strengthening muscles helps manage sarcopenia and osteoarthritis, while weight-bearing activities help maintain bone density in osteoporosis and osteopenia. In nutrition, the focus in the art is on adequate intake of calcium, vitamin D and protein. In some cases, medications such as bisphosphonates (for osteoporosis/osteopenia) and antiinflammatory drugs (for osteoarthritis) are used.

There is a continuing need for ways to provide a reduction in the risk of development of medical musculoskeletal degenerative disorders, or to treat such disorder after it has manifested itself. There is in particular a need for a way that can be used to supplement known options of such treatment, for instance with the aim to address mobility problems in a subject in need thereof.

The present inventors have found that a combination of different bioactives, at least one of which is a biotic, addresses this need.

Accordingly, the present invention relates to a pharmaceutical or nutraceutical composition comprising (a combination of) at least a first bioactive, which is a biotic, and at least a second bioactive, which may be a biotic or another bioactive, wherein said first bioactive and said second bioactive are different from each other, in particular of a different type, more in particular of a different category.

The invention further relates to a combination of at least a first bioactive and at least a second bioactive, wherein said first bioactive and said second bioactive are different from each other, in particular of a different type, more in particular of a different category, for use in the treatment of a medical musculoskeletal degenerative disorder. Herein, said first and said second bioactive are active components, at least in combination, for use in said treatment, which treatment may be a prophylactic treatment, or a treatment of a subject having the medical disorder.

When referring to a combination for use in accordance with the invention, at least said first and said second bioactive are typically both present (and typically for use) as an active component for the intended use. Thus, the phrase 'combination (of at least a first bioactive and at least a second bioactive) for use in' can also be read as: 'at least a first bioactive and at least a second bioactive for use in combination in ...'. Generally, the active ingredients (or at least the at least first and the at least second bioactive in a combination for use in accordance with the invention form part of the same composition, which may be a pharmaceutical or nutraceutical composition.

Said first bioactive is typically a biotic selected from one of the following categories of biotics: probiotics, parabiotics, postbiotics and prebiotics. Said second bioactive is typically selected from one of the following categories of biotics: probiotics, parabiotics, postbiotics, prebiotics and further (joint-supportive) bioactive compounds.

### Brief description of the drawings

Abbreviations in the figures: OA, osteoarthritic; Vit C, vitamin C; Col, collagen; CS, chondroitin sulphate; GS, glucosamine; CM, Chondro Mix (Chondroitin Sulphate + glucosamine); KP: peptides from kefir-fermented milk; EPA: collagen mix + hyaluronic acid + vitamins B1, B2, B6, C, E, silicium and magnesium; NEM, egg-shell membrane; Turm: turmeric acid; mangosteen ext.: combination of xanthones derived from mangosteen extracts; 3SL: 3'-syalyllactose, 6SL: 6'-syalyllactose, Calanus oil: calanus oil rich in omega-3 fatty acids; CBD: cannabidiol; Resolvin: specialized pro-resolving mediator derived from omega-3 fatty acids; Pimp: Pimpinella extract; VFP46: live *Lactiplantibacillus plantarum* VFP46 (probiotic); LZT: larazotide; ABB i33: ABB i33 yeast extract from Torula, ABB S1: *Saccharomyces boulardii* ABB S1, ABB S7: *Kluyveromyces marxianus* ABB S7, ABB S32: *Saccharomyces cerevisiae* ABB S32, ABB i33, ABB S1, ABB S7 and ABB S32 are exemplary biotics, obtainable from ABbiotek Health (Barcelona, Spain, http://abbiotekhealth.com).
Figure 1: Effects of the different treatments and combinations in chondrocytes viability (Figure 1A) and proliferation, 5-Bromo-2'-deoxyuridine (BrDU) assay (Figure 1B) in conditions of osteoarthritis in the presence of the different treatments and combinations. Data are mean ± SEM. ***p*<0.01, ****p*<0.001, *****p*<0.0001.
Figure 2: Evaluation of caspase activation (Fig. 2A) and the percentage of early (Fig. 2B) and late (Fig. 2C) apoptotic chondrocytes in conditions of osteoarthritis in the presence of the different treatments. Data are mean ± SEM. **p*<0.05 *vs.* osteoarthritis (OA) vehicle.
Figure 3: Evaluation of chondrocyte *Colla1* (Fig. 3A) and *Col2a1* (Fig.3B); gene expression in conditions of osteoarthritis in the presence of the different treatments and combinations. Data are mean ± SEM. **p*<0.05; **p<0.01; *****p*<0.0001 *vs*. osteoarthritis (OA) vehicle.
Figure 4: Evaluation of chondrocyte *Col10a1* (Fig. 4A) and *SOX9* (Fig.4B) gene expression in conditions of osteoarthritis in the presence of the different treatments and combinations. Data are mean ± SEM. **p*<0.05; ***p*<0.01; *****p*<0.0001 *vs*. osteoarthritis (OA) vehicle.
Figure 5: Evaluation of chondrocyte *ECM1* gene expression in conditions of osteoarthritis in the presence of the different treatments and combinations. Data are mean ± SEM. **p*<0.05; ***p<0.01;* ****p*<0.001; *****p*<0.0001 *vs*. osteoarthritis (OA) vehicle.
Figure 6: Evaluation of chondrocyte *MMP1* (A); *MMP13* (B) and *TIMP1* (C) gene expression in conditions of osteoarthritis in the presence of the different treatments and combinations. Data are mean ± SEM. **p*<0.05; ***p*<0.01; ****p*<0.001; *****p*<0.0001 *vs*. osteoarthritis (OA) vehicle.
Figure 7: Evaluation of chondrocyte *AGC1* (A) and *GBL* (B) gene expression in conditions of osteoarthritis in the presence of the different treatments and combinations. Data are mean ± SEM. **p*<0.05; ***p*<0.01; ****p*<0.001; *****p*<0.0001 *vs*. osteoarthritis (OA) vehicle.
Figure 8 shows creatinase activity of a number of combinations of biotics according to the invention in a sarcopenia model. Data are mean ± SEM. **p*<0.05; ***p*<0.01; ****p*<0.001; *****p*<0.0001 *vs*. Sarcopenic vehicle.
Figure 9 shows beta-galactosidase activity of a number of combinations of biotics according to the invention in a sarcopenia model. Data are mean ± SEM. **p*<0.05; ***p*<0.01; ****p*<0.001; *****p*<0.0001 *vs*. Sarcopenic vehicle.
Figure 10 shows the percentage of myoblasts in S phase of a number of combinations of biotics according to the invention in a sarcopenia model. Data are mean ± SEM. **p*<0.05; ***p*<0.01; ****p*<0.001; *****p*<0.0001 *vs*. Sarcopenic vehicle.
Figure 11 shows the percentage of early apoptotic cells of a number of combinations of biotics according to the invention in a sarcopenia model. Data are mean ± SEM. **p*<0.05; ***p*<0.01; ****p*<0.001; *****p*<0.0001 *vs*. Sarcopenic vehicle.
Figure 12 shows alkaline phosphatase activity of a number of combinations of biotics according to the invention in a osteopenia model. Data are mean ± SEM. **p*<0.05; ***p*<0.01; ****p*<0.001; *****p*<0.0001 *vs*. osteoporotic vehicle.
Figure 13 shows beta-galactosidase activity of a number of combinations of biotics according to the invention in a osteopenia model. Data are mean ± SEM. **p*<0.05; ***p*<0.01; ****p*<0.001; *****p*<0.0001 *vs*. osteoporotic vehicle.
Figure 14 shows BrDU incorporation of a number of combinations of biotics according to the invention in a osteopenia model. Data are mean ± SEM. **p*<0.05; ***p*<0.01; ****p*<0.001; *****p*<0.0001 *vs*. osteoporotic vehicle.
Figure 15 shows caspase activation of a number of combinations of biotics according to the invention in a osteopenia model. Data are mean ± SEM. **p*<0.05; ***p*<0.01; ****p*<0.001; *****p*<0.0001 *vs*. osteoporotic vehicle.
Figure 16: Evaluation of *Bglap* gene expression in conditions of osteoarthritis in the presence of the different treatments and combinations. Data are mean ± SEM. **p*<0.05; ***p*<0.01; ****p*<0.001; *****p*<0.0001 *vs*. osteoarthritis (OA) vehicle.

For the purpose of clarity and a concise description, features are described herein as part of the same or separate embodiments, however, it will be appreciated that the scope of the invention may include embodiments having combinations of all or some of the features described.

### Definitions

As used herein, the singular forms "a", "an" and "the" are intended to include the plural forms as well. The term "or" includes any and all combinations of one or more of the associated listed items, unless the context clearly indicates otherwise (e.g. if an "either ....or" construction is used).

It will be understood that the terms "comprises" and "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise.

In the context of this disclosure, the term "about" means generally a deviation of 15 % or less from the given value, in particular a deviation of 10% or less, more in particular a deviation of 5% or less.

When referring to substances (e.g. matter, compounds, ions) in the present disclosure which are an acid or a base, the conjugated base respectively conjugated acid is meant to be included when referring to these substances or other components. E.g. when referring to chondroitin or glucosamine, these terms include salts thereof, like chondroitin sulphate respectively glucosamine sulphate.

As is used herein, the term "inactivated", or "dead", or "non-viable", refers to an organism, such as a yeast, not being capable of reproduction or colonization. An inactivated organism can have intact or broken cell membranes. The skilled person will be able to obtain an inactivated organism yeast based on common general knowledge and the information disclosed herein. Possible means include irradiation, heat inactivation, sonication, lyophilization and chemical inactivation. An example of chemical yeast inactivation is an inactivation with Thimerosal, i.e. sodium ethyl mercury thiosalicylate, which is able to inactivate yeasts and bacteria.

As used herein, the term "heat-killed", refers to an organism inactivated by heat-treatment and such not capable of metabolic activity nor colonization. Means of heat-treatment to inactivate an organism are known to a person skilled in the art and include tyndallization, pasteurization, ultra-high temperature (UHT) heating, Ohmic heating (or Joule heating), blanching, drying, boiling and sterilization.

As is used herein, the term "tyndallization", refers to a sterilization process often used to heat-kill probiotic microorganisms. Tyndallization involves repeating a heat-killing step for certain consecutive days, for example as described on page 11 in the Handbook of Microbiological Media (third edition, 2004, CRC Press) by Ronald M. Atlas. Therefore, the term "tyndallized", refers to an organism having been heat-killed by tyndallization and such not capable of metabolic activity or colonization.

As is used herein, the term "alive", or "viable", refers to an organism capable of reproduction or colonization.

As used herein, the term "cell lysis" refers to any type of cell disruption that results in the release of intercellular biological components naturally contained in the cells of an organism. Therefore, the term "lysate", refers to a product obtained after cell lysis. A "lysate", as used herein, means in particular essentially the entire lysate obtained by lysis of an organism and therefore comprises macromolecules such as DNA, RNA, proteins, peptides and lipids from the lysed cell; as well as cellular debris, including cell wall material, and cell membrane components from the lysed cells. Methods for obtaining a lysate are known to a person skilled in the art and include enzymatic, physical and chemical methods. Cell wall components can be separated from the fluid part of the lysate e.g. by centrifugation of filtering.

As used herein, the term "extract" of a microorganism, refers to a fluid part or fraction of the microorganism cell or lysate, in particular liquid contents of yeast or bacterial cells, in particular obtainable by filtration or by centrifugation, or a fraction thereof, obtainable by extraction from the cells or the lysate, using an extracting phase.

As used herein, the term "metabolite", refers to any substance derived from the growth or maintenance of the yeast, persisting in the culture medium and with no need to be preserved by special techniques. Examples of metabolites are organic and inorganic acids, proteins, (poly)peptides, amino acids, (co)enzymes, fatty acids, (esterified) lipids, carbohydrates (including monosaccharides, disaccharides and polysaccharides), lipoproteins, glycolipids, glycoproteins, sugar phosphates, vitamins, salts, metals, or nucleic acids.

As is used herein, the term "alive", or "viable", refers to an organism being capable of reproduction or colonization.

As is used herein, the term "individual", refers to any living organism such as an animal or human that can benefit from the administration of a component or a physiologically accepted composition for use according to the invention. The term "animal" as used herein, refers in particular to vertebrate animals including fish, birds, mammals, reptiles and amphibians. The animal can be a farm animal, domestic animal or laboratory animal. An individual, which may be treated in accordance with the invention may in particular be selected from humans, nonhuman primates and monkey species, cows, sheep, pigs, goats, horses, dogs, cats, rodents such as mice, rats and guinea pigs, poultry, such as chickens, hens, turkeys, ducks and geese and aquatic animals such as fish and shrimp. The term individual does not denote a particular age or sex (such as male/female). Thus, humans of any age group, including adults (older than 18 years of age) and children (0-18 years of age), e.g. newborn individuals (0-12 months of age), toddlers (12-36 months of age), can be treated in accordance with the invention. In a preferred embodiment, the component or the composition is for use in the treatment of a human.

As used herein, a "derivative strain" is a strain derived from a strain disclosed herein, including through hybridization, mutagenesis, recombinant DNA technology, mating, cell fusion, or cytoduction between strains. Further, derivative strains, in particular derivative strains of bacteria, may be obtained by transformation, transduction or conjugation. A derivative strain can be a strain found in nature (a natural strain), yet isolated from its natural environment. In an embodiment, the derivative strain is not found in nature (a non-natural strain). Advantageously, the derivative strain is a progeny strain of a parent strain (i.e. a strain from which it is a progeny), which progeny strain typically is the product of a mating between said parent strain and another parent strain or the progeny strain is a descendent of that mating product. Preferably, the progeny strain can be obtained using a hybridization method comprising (i) hybridization of a parent strain mentioned in the present disclosure, or a derivative of any of these and (ii) at least one step of screening or selection of the hybrid obtained, which selection is based on one or more defining characteristics of said parent strain. For instance, a defining characteristic can be based on taxonomic classification. Further. A defining characteristic can be based on a parameter/marker as identified herein, in particular in the examples, as relevant in the treatment of a medical musculoskeletal degenerative disorder.

In the context of the present invention, a **"biotic"** refers to a category of compounds that influence the gut microbiome, overall health or both. A biotic is typically naturally occurring or originating/obtained from a naturally occurring being. Generally, a biotic is a micro-organism or matter obtained or derived from a micro-organism, such as a part thereof (e.g. extract, cell wall component, lysate) or a metabolite thereof. Notably prebiotics, in particular prebiotic saccharides, may be obtainable from a multi-cellular organism, such as a plant, or from mammalian milk. Biotics usually support overall health of an multicellular organism, such as a human or animal, by supporting the microbial balance in the digestive system. In accordance with the present invention, without being bound by theory, it is considered that the biotic can reinforce the gut barrier function to reduce systemic inflammation, acting on the gut-joint axis as a prevention of cartilage / muscle / bone degradation.

The term "bioactive" generally refers to matter (substances) that interact with or benefit living organisms. The bioactive can be a naturally occurring matter (including parts thereof, such as lysates, extracts, cell wall components, metabolites of cells etc., which may be obtained by a physical process) or a synthetic matter (not-naturally occurring, requiring a (bio)chemical synthesis). Examples of synthetic bioactives are synthetic joint-supportive peptides (e.g. larazotide) and synthetic polyunsaturated fatty acid derivatives and SPMs (e.g. resolvins). Bioactives from naturally occurring matter can be obtained or derived from a plant (e.g. botanical extracts), matter obtained or derived from an animal (e.g. chondroitin sulphate, some types of collagen, calanus oil), matter obtained or derived from a fungus (which may be a micro-organism or not), or matter obtained or derived from a protist (which protist may be a micro-organism or not).

Probiotics are live microorganisms (typically beneficial bacteria or beneficial yeasts) that, when consumed in adequate amounts, provide health benefits to the host. Examples of suitable probiotics include lactic acid producing bacteria, such as *Bifidobacteria, Lactobacilli, Leuconostoc, Pediococcos,* and probiotic yeasts, such as probiotic *Saccharomyces,* probiotic *Kluyveromyces.* Good results are in particular achieved with *Bifidobacteria, such as B. bifidum or B. longum, with Lactobacilli, such as L. plantarum,* and with *Kluyveromyces marxianus.*

Prebiotics: Prebiotics are substrates selectively utilized by host microorganisms that confer a health benefit to the host (Nature Reviews Gastroenterology & Hepatology volume 14, pages491-502 (2017)). Typically, prebiotics are at least partially fermentable by a microbiome. Well-known prebiotics are digestible or not fully digestible saccharides that promote the growth of beneficial microorganisms, primarily in the gut; these include fructo-oligosaccharides; galacto-oligosaccharides; mannanoligosaccharides, xylooligosaccharides; and human milk oligosaccharides (HMO), such as sillyllactose 3SL and sillyllactose 6SL. Non-saccharide prebiotics include prebiotic polyunsaturated fatty acids, like conjugated linoleic acids; and prebiotic polyphenols.

Postbiotics are preparations of inanimate microorganisms, fractions thereof or components thereof that confer a health benefit on the host (Salminen S, Collado MC, Endo A, Hill C, Lebeer S, Quigley EMM, et al. The International Scientific Association of Probiotics and Prebiotics (ISAPP) consensus statement on the definition and scope of postbiotics. Nat Rev Gastroenterol Hepatol. 2021;18(9):649-67). These components include short-chain fatty acids, vitamins, enzymes, antimicrobial peptides, macromolecular complexes of biomolecules, such as complexes of lipoproteins and (oligo)saccharides. Fractions thereof include extracts, cell wall components and lysates. When postbiotics are inactivated or dead bacterial whole cells that may still exert health benefits even though they are no longer alive, they are also called parabiotics, "non-viable probiotics", "tyndallized probiotics", "heat-treated probiotics" or "ghost probiotics". A postbiotic can be a component or fraction obtained from a probiotic or be inactivated/dead whole cells of a probiotic , but it is also possible to obtain a postbiotic from a micro-organism that is not probiotic as such.. A component produced by a microorganism can also be a postbiotic. Preferred postbiotics include *Bifidobacteria* lysates, such as *B. bifidum* lysates or *B. longum* lysate; yeast extracts, such as Torula (*Cyberlindnera jadinii*) extracts; peptidic post-biotics, such as peptides from Kefir-Fermented Milk Powder. It is not necessary to isolate such peptides from the kefir; it also possible to include kefir-fermented milk powder in the combination or composition. If desired the peptides can be isolated from the Kefir. This can be done in a manner known per se. Particularly useful joint supportive peptides are considered to have a molecular weight of less than about 3000 g/mol..

Useful (joint-supportive) bioactive ingredients as a second or further bioactive of a combination or composition of the invention include (joint-supportive) botanical extracts; (joint-supportive) omega-3 polyunsaturated fatty acids; (joint supportive) glycosamine (GAG) system components (including GAGs and precursors for GAGs); (joint supportive) amino acids, peptides and proteins; (joint supportive) vitamins.

Joint supportive botanical extracts include mangosteen extract, cat's claw extract (*Uncaria tormentosa*) and *Pimpinella* extract, such as *P. anisum* extract. Further examples of (joint supportive) bioactives that may be included as a second or further active include turmeric acid, cannabidiol (CBD), xanthones (e.g. obtainable from mangosteen).

Joint supportive GAG system components include chondroitins, glucosamines (chondroitin precursor), dermatan, keratan, hyaluronic acid.

Joint supportive proteins, peptide and amino acids include collagen, elastin, desmosin (cross-linking amino acid), kefir peptides, larazotide. The collagen or elastin may be a hydrolysate.

Joint supportive omega-3 polyunsaturated acid sources include resolvins and calanus oils.

In particular good results have been achieved with a joint supportive ingredient selected from chondroitins, glucosamins, collagens (which may be intact or a hydrolysate), hyaluronic acids, dermatans, keratans, desmosines, and elastins. Said joint-supportive products can e.g. be obtained from egg-shell membrane, or egg-shell membrane can be included in the combination to provide said products. Alternative sources for these components are well-known in the art. E.g. they can be extracted from an animal source or produced microbiologically using a (genetically modified) yeast or bacterium. In particular chondroitin can be obtained from animal cartilage, glucosamine from crustacean shells.

### Detailed description

A bioactive in a composition or in a combination for use according to the invention can be included in a purified form or as part of a composition. E.g. a post-biotic component prepared fermentatively by a micro-organism can be included isolated from the medium wherein it has been produced, or as part of the, optionally dried, medium wherein it is produced, or a fraction thereof. E.g., a fermented milk (or powder thereof), such as kefir-fermented milk (powder) is a preferred source for a peptidic post-biotic.

Two or more different bioactives in a composition of in a combination for use in accordance with the invention can be of a different category, or they can be of the same category (in particular both postbiotic), but of a different type, e.g. both a post-biotic but one is an extract and the other is a lysate. The first and the second biotic are typically of a different species though. If one of the first biotic and second biotic is a probiotic, the other is generally selected from parabiotics, postbiotics, prebiotics and further (joint-supportive) ingredients. If one of the first biotic and second biotic is a prebiotic, the other is generally selected from parabiotics, postbiotics, probiotics and further joint-supportive ingredients. The first bioactive and the second bioactive are generally from a different source. I.e. if the first bioactive and the second bioactive are both a microbiological matter (which may be probiotic or postbiotic) the first bioactive is from a different species than the second bioactive.

One or more biotics, preferably two or more biotics are advantageously chosen from the group of lactic acid producing bacteria lysates, more preferably *Bifidobacterium,* lysates, in particular *B. longum* lysates; yeast extracts comprising nucleotides and two or more vitamins selected from the group of B1 (thiamine), B2 (riboflavin), B3 (niacin), B5 (pantothenic acid), B6 (pyridoxine), B7 (biotin), B9 (folic acid), and B12 (cobalamin), more preferably Torula yeast extracts; peptidic postbiotics, more preferably kefir peptides, which may be isolated or be part of a fermented product, e.g. dried fermented product (e.g. Kefpep^{®}); viable probiotic yeasts, more preferably *K. marxianus.* A prebiotic, such as a sillyllactose, preferably sillyllactose 3SL, sillyllactose 6SL or both, is advantageously used as a second bioactive, in particular for use in the treatment of osteoarthritis.

A highly preferred biotic is a *B. longum,* biotic, which may be a probiotic or a postbiotic. A combination comprising such a biotic, in particular a lysate, has been found effective in models for several medical disorders, notably, sarcopenia and osteoarthritis. In particular good results have been achieved with a composition or combination according to the invention comprising *B. longum CBi0703* (post)biotic, especially a lysate of *B. longum CBi0703.* A representative sample of *B. longum* Cbi0703 has been deposited, w *B. longum CBi0703,* with the Collection Nationale de Cultures de Microorganismes (CNCM) at the Institut Pasteur in Paris, under the accession number CNCM I-3994. Thus, in a highly preferred embodiment, at least one of the biotics is a (post)biotic of a *B. longum CBi0703* or a *B. longum Cbi0703* derivative strain (post)biotic, in particular a lysate of *B. longum CBi0703* or a lysate of a *B. longum CBi0703* derivative strain. In particular suitable derivative strains are progeny strains of B. *longum CBi0703.*

In an advantageous embodiment, the *B.longum CBi073* postbiotic comprises a bacterial macromolecular complex, consisting of chains combining a lipoprotein and an oligosaccharide, characaterised in that:
the lipoprotein has a molecular weight of from 30 kDa to 60 kDa;
the oligosaccharide has a molecular weight of less than 15 kDa, and preferably less than 10 kDa;
the lipoprotein component, which consists of all the lipoproteins of each of the chains, represents from 75 to 99%, preferentially from 80 to 98%, more preferentially from 85 to 95% by weight of the total weight of the complex, and the oligosaccharide component, which consists of all the oligosaccharides combined with each of the chains, represents from 1 to 25%, preferentially from 2 to 20%, and more preferentially from 5 to 15% of the total weight of the complex. Such complex can be obtained as described in
US2013/130988 A1, of which the contents - in particular the claims 1-20, the sequence identities of the lipoproteins and the Examples are incorporated herein by reference.

The amounts of the at least first and the at least second bioactive in a composition according to the invention or a combination for use according to the invention can be chosen in a broad range. The skilled person will be able to determine an effective amount and dose based on the information disclosed herein, e.g. the Examples and common general knowledge. Where an individual bioactive is already known in the art for use in a certain treatment, known amounts and doses may be taken into consideration as well. Generally, the weight to weight ratio of the first bioactive (or first type or category of bioactives) to the second bioactive (or first type or category of bioactives) may be chosen in the range of 1:1000 to 1000:1, preferably in the range of in the range of 1:100 to 100:1, more preferably in the range of 1:50 to 50:1, in particular in the range of 1:25 to 25:1, more in particular in the range of 1:10 to 10:1, more in particular in the range of 1:1 to 10:1 or in the range of 10:1 to 1:1, even more in particular in the range of 1:1 to 5:1 or in the range of 5:1 to 1:5.

For effective combinations or compositions shown in the Examples, one may in particular choose a ratio of first bioactive type to second bioactive type that is up to 25 times higher or up to 25 times lower than the demonstrated effective combination, more in particular in the range of up to 10 times lower to up to 10 times higher, even more in particular of up to 3 times lower to up to 3 times higher,

For applications wherein the combination or composition includes B. *longum* (post)biotic, advantageously a *B. longum* lysate, a ratio in the range of 1:10 - 1:25 *B. longum* to the total of second and optionally further joint-supportive bioactives , is highly preferred for its effectivity in the treatment of a medical disorder in accordance with the invention.

A composition or combination according to the invention may be for use in the treatment of a variety of musculoskeletal degenerative disorder, in particular including a medical disorder selected from the group of sarcopenia, osteoporosis, osteopenia and osteoarthritis.

In a preferred embodiment, the combination is for use in the treatment of sarcopenia. In particular good results have been achieved in the treatment of sarcopenia, comprising biotics as the first bioactive and the second bioactive, more in particular with a combination for use according to the invention wherein said first bioactive is *a Bifidobacterium* lysate, and said second bioactive is a yeast extract, as illustrated in Example 2. A preferred lysate for use in the treatment of sarcopenia is a *B. longum* lysate. A preferred yeast extract is Torula yeast extract. This yeast extract provides a significant amount of nucleotides and vitamins B, which is considered advantageous in the treatment. In a combination for use in the treatment of sarcopenia, the weight to weight ratio of the first biotic, in particular when including the *B. longum* postbiotic, advantageously lysate (such as a lysate of *B. longum Cbi0703* or of a derivative strain thereof) to the second biotic, in particular when including yeast extract (advantageously Torula) for use in the treatment is usually in the range of 1:100 to 100:1, preferably in the range of 1:50 to 50:1, in particular in the range of 1:25 to 25:1 more in particular in the range of 1:5 to 5:1. In particular good results have been achieved with a combination comprising about equal amounts of *B. longum* lysate and Torula yeast extract.

In a further preferred embodiment, the combination is for use in the treatment of osteoporosis or osteopenia. For use in such treatment, it is particularly preferred to include a peptidic bioactive, such as a peptidic post-biotic in the combination. A highly preferred post-biotic is a post-biotic comprising kefir peptides. A preferred second bioactive is a yeast extract, of which Torula yeast extract is highly preferred. This yeast extract provides a significant amount of nucleotides and vitamins B, which is considered advantageous in the treatment. It is further advantageous to include a further biotic, such as a viable yeast. A preferred viable yeast is *K. marxianus.*

Advantageously the weight to weight ratio of the first bioactive, in particular when including a bioactive product comprising a joint-supportive peptide (advantageously comprising kefir peptides) to the second bioactive (advantageously including yeast extract, such as Torula extract) for use in the osteoporosis or osteopenia treatment is usually in the range of 1:1000 to 1000: 1, preferably in the range of 1:250 to 250:1. When including the biotic peptides in isolated form, the content of biotic peptides to yeast extract may in particular be at least 1:250, more in particular in the range of 1:125 to 1:10. When including biotic peptides as part of a at least substantially complete fermentation product (such as fermented milk, kefir), the content of the complete fermentation product (based on dry weight) may in particular be in the range of 1:10 to 10:1, more in particular in the range of 1:5 to 5:1. In particular good results have been achieved with a combination comprising about equal amounts of dried kefir and Torula yeast extract.

In a further embodiment, the combination is for use in the treatment of osteoarthritis. For use in the treatment of osteoarthritis, the combination preferably comprises a *Bifidobacterium* biotic, preferably a *Bifidobacterium* post-biotic more preferably a *Bifidobacterium* lysate, as a first biotic. Good results have been achieved with a *B. longum* postbiotic, more in particular *B. longum* lysate, such as a *B. longum* Cbi0703 lysate or a lysate of a derivative strain thereof. For use in the treatment of osteoarthritis, the combination may advantageously comprise further comprise a prebiotic as a bioactive, in particular a sialyllactose, more preferably sillyllactose 3SL, sillyllactose 6SL or both, even more preferably both.

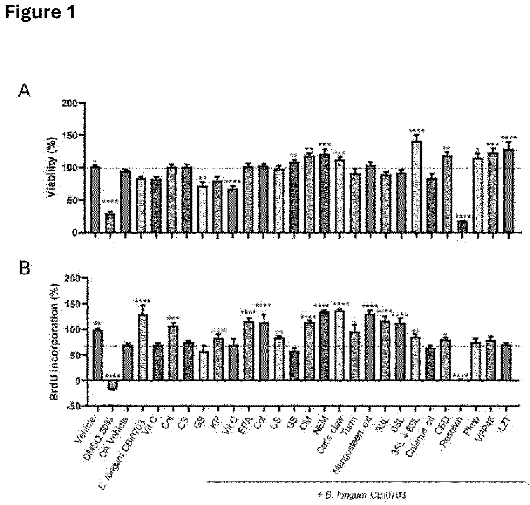

Alternatively or in addition for use in the treatment of osteoarthritis, the first bioactive is a biotic (such as the *B. longum* biotic mentioned in the previous paragraphs), in particular a probiotic or postbiotic, which is is combined with one or more biotic joint-supportive compounds selected from the group of chondroitins, glucosamines, collagens, hyaluronic acids, dermatans, keratans, desmosines, and elastins. Particularly good results have been achieved with one or more of collagen, glucosamine, and chondroitin. Advantageously, egg-shell membrane is included in the combination or composition of the invention, which is a good source for each of collagen, glucosamine and chondroitin. As supported in Example 1 (Figure 1A), when compared to a combination of *B. longum* biotic alone or *B. longum* biotic plus vitamin C, the combination of the *B. longum* biotic with (1) glucosamine, (2) chondroitin+glucosamine, or (3) egg shell membrane induces significantly improved cell growth and proliferation in osteoarthritis. Said three combinations of the invention also contribute to preventing cartilage degradation as observed in chondrocyte proliferation (Figure 1b), an effect also illustrated for (4) the combination of the invention with collagen.

In a composition according to the invention or combination for use in the treatment of osteoarthritis according to the invention, the weight to weight ratio of the first bioactive, in particular a probiotic or postbiotic, in particular when including the *B. longum* postbiotic, to the total of said biotic joint-supportive compiunds for use in the treatment is usually in the range of 1:500 to 500:1 in particular in the range of 1:250 to 250:1, more in particular in the range of 1:100 to 100:1, more in particular in the range of 1:25 to 10:1 more in particular in the range of 1:10 to 5:1. Preferred ratio's may be based on known dosages for individual components. In particular good results are achieved with a composition or combination comprising *B. longum* postbiotic and one or more of collagen, chondroitin and glucosamine, preferably comprising both chondroitin and glucosamine, wherein the ratio of the *B. longum* postbiotic to the total of collagen, chondroitin and glucosamine is in the range of about 1:1 to about 1:400, more preferably 1:30 to 1:250. E.g. *B.longum postbiotic* to collagen ratio may advantageously be about 1:100 to about 1:300, in particular about 1:200; *e.g. B.longum postbiotic* to chondroitin ratio may advantageously be about 1:32 to about 1:48; e.g. *B.longum postbiotic* to glucosamine ratio may advantageously be about 1:40 to about 1:60. In particular when using egg-shell membrane, a relative low amount of the egg-shell membrane is already highly effective. This suggests an extra synergistic effect of the plurality of joint-supportive compounds in combination with the *B. longum* postbiotic. In a preferred embodiment, the ratio *B. longum* postbiotic to egg-shell membrane is in the range of 1:1 to 30:1, in particular in the range of about 1:1 to about 20:1.

### Administration in a treatment in accordance with the invention

The composition or the combination for use according to the present invention is usually administered into the gastrointestinal tract, e.g. orally. In a specific embodiment, the composition is administered by tube feeding or as a suppository. Formulations of a composition of the invention or a combination for use according to the invention suitable for oral intake include but are not limited to: capsules, coated capsules, tablets, sachets, pills, pearls, soft-gels, vials, powders, granules, solutions, suspensions, emulsions, elixirs, syrups, sprays, lozenges, troches, gums, hard candies and gels.

In a specific embodiment, the combination or composition is to be administered as a sustained release product. The composition or combination may may be a fermented food product or a non-fermented food product. Examples of a particularly suitable food products include dairy products such as a yogurt, a yogurt drink, cheese, milk, milk powder, infant formula, cream, ice-cream, cream powder and butter; fruit-based products such as fruit juice, compote or fruit jelly; solid foodstuffs such as flours, cereals, a snack, a biscuit, and liquid formulations such as vegetable beverages, smoothies, isotonic drinks, salts solutions and enteral nutrition recipes.

The composition or combination may be in the form of a dietary supplement, a food additive, a feed additive, a functional food in human nutrition, a functional food in animal nutrition, a food additive or functional ingredient for nutraceuticals or food for special medical purposes.

The composition or combination can be a pharmaceutical product or a nutraceutical product. Said pharmaceutical product may further comprise a pharmaceutically acceptable adjuvant and/or excipient. Adjuvants and excipients are well known to a person skilled in the art.

If desired, a bulking agent can be included, in particular a carbohydrate, for example maltodextrin.

The administration dosage, duration and frequency can be chosen within wide limits, dependent on the intended purpose and the subject to whom the composition is to be administered. The duration of treatment can be a relatively short period, e.g. of a week or less, or a day or less, e.g. for an acute manifestation of a disorder or symptom of a disorder. The duration of a treatment can also be prolonged, e.g. for a week or more, a month or more or a year or more, e.g. in case of a chronic disorder or in case of preventive treatment or dietary supplementation.

A person skilled in the art will be able to determine an appropriate dose of the component or physiologically acceptable composition to administer to an individual based on common general knowledge and the information disclosed herein without undue experimentation. If an individual biotic is already known in the art for a certain treatment, public knowledge about dosages for such biotic may also be taken into consideration. As the skilled person will understand, an actual preferred dosage depends on a variety of factors, including the age, body weight, general health, sex and species of the individual diet, mode and time of administration, rate of excretion, drug combination, the severity of the particular disorder, and the individual.

The dosages disclosed herein are indicative of an average case for a human individual (including adults and children). There can of course be individual instances where higher or lower dosage ranges are merited.

As a rule of thumb, a daily dosage for a bacterial biotic (notably *B*. *longum* biotic), in particular a postbiotic, such as a lysate, may be chosen in the range of 1-100 mg, 5-75 mg, 10-50 mg or 15-40 mg. E.g. for *B. longum* (post)biotic, such as lysate, a daily dosage of about 25 mg is found highly advantageous in a clinical setting.

As a rule of thumb, for kefir fermented milk powder (e.g. Kefpep^{®}), providing joint supportive peptide, a daily dosage may advantageously be chosen in the range of about 0.8 to about 2.0 g/day. In particular for a prophylactic purpose or patient having only mild symptoms of osteopenia, the dosage may advantageously be chosen in the range of 0.8-1.0 g/day. For an osteoporosis patient or severe osteopenia patient a daily dosage of 1.6-2.0 g is recommended.

As a rule of thumb for yeast probiotics and yeast postbiotics a daily dose may be chosen in the range of about 20 to about 1000 mg, preferably in the range of about 50 to about 500 mg.

As a rule of thumb, a recommended daily dosage for collagen, for instance in combination with a B longum (post)biotic is 1-10 g, in particular 5-10 g.

As a rule of thumb, a recommended daily dosage for chondroitin, for instance in combination with a B longum (post)biotic is 500-1000 mg, in particular 800-1000 mg.

As a rule of thumb, a recommended daily dosage for glucosamine, for instance in combination with a B longum (post)biotic is about 500 to about1500 mg, in particular about 1000 to about 1500 mg.

The invention further relates to larazotide for use in the treatment of a medical mobility disorder or a medical musculoskeletal degenerative disorder, preferably a medical disorder selected from the group of sarcopenia, osteopenia, osteoporosis and osteoarthritis.

The invention provides a method for treating an individual in need thereof, comprising administering an effective amount of the combination for use in accordance with the invention or the larazotide in accordance with the invention.

The invention provides the use of the first bioactive and the second bioactive for use in the preparation of the pharmaceutical composition, the nutraceutical composition or the combination, for use in the treatment of the degenerative skeletomuscular disorder.

A method of treatment or combination for use or the use according to the invention can be prophylactic with respect to the skeletomuscular medical disorder or be the treatment of a subject having the skeletomuscular disorder. The treated subject in a method or combination for use according to the invention The invention provides a method respectively combination for use of treating an individual having the skeletomuscular medical disorder or having a genetic disposition to developing said medical disorder.

The effectivity of a prophylactic treatment can routinely be demonstrated, e.g. by monitoring the incidence of a disorder becoming manifesting in a cohort study wherein the incidence in a group of subjects treated with a composition according to the invention is compared with the incidence in a reference group of subjects (e.g. treated with placebo). The effect of a treatment of an individual having the medical disorder can be a complete cure, an alleviation of a symptom, reduced suffering etc.

The subject in a combination for use, in a method of treatment or the use of the combination is typically a human, preferably an adult human, in particular human of the age of 50 years or more, 60 years or more or 65 years or more.

### The production of a composition or combination in accordance with the invention

The composition or the combination for use according to the invention can be produced by blending the different components. Optionally, one or more other components may be included in the composition in addition to said bioactives, e.g. components for the composition mentioned elsewhere herein. In particular, one or more further components may be added selected from the group of additives, excipients, proteins, vitamins, carbohydrates, minerals.

The bioactives may be prepared in a manner known per se. In an advantageous embodiment, the production of a postbiotic comprises heat-killing of a micro-organism , such as tyndallization. The micro-organism may but does not have to be a probiotic, as long as the heat-killed organism is a biotic.

The invention will now be illustrated by the following examples, which are provided by way of illustration and it will be understood that many variations in the methods described and the amounts indicated can be made without departing from the spirit of the invention and the scope of the appended claims.

### Example 1 Osteoarthritis treatment

### (B. longum post-biotic plus joint-supportive compound)

### Materials and methods

### Cell culture conditions

To investigate osteoarthritis (OA) mechanisms and potential treatments, Chondrosarcoma cell lines, such as the human chondrosarcoma cell line, SW1353 are commonly used as surrogates for primary chondrocytes due to their chondrogenic properties and reproducible response to OA-related stimuli. SW1353 was obtained from the American Type Culture Collection (ATCC, Manassas, VA, USA) and cultured in Leibovitz's L-15 Medium (L15) containing 10 % (v/v) fetal bovine serum (FBS) and antibiotics (v/v) (penicillin-streptomycin). Cells were maintained at 37°C in a humidified 0 % CO₂ atmosphere.

The *B. longum CBi0703* post-biotic (lysate) was obtained from the freeze-dried culture of *Bifidobacterium longum* CBi0703 by grinding. The result of this process is a mixture of *B. longum* metabolites and cell fractions, including DNA, together with components of the culture medium.

The following table includes the ingredients and combinations evaluated in our OA model.

**Table 1:**

| **Test** | **Name of combination** | **Experimental dose** |
|---|---|---|
| *CE1* | *B. longum CBi0703* | 200 µg/mL |
| Control | Vitamin C (Vit C) | 200 µg/mL |
| Control | Collagen (Col) | 1 mg/mL |
| Control | Chondroitin sulphate (CS) | 1 mg/mL |
| Control | Glucosamine sulphate (GS) | 1 mg/mL |
| | *B. longum* CBi0703+ Kefir fermented milk peptide product (KP) | 200 µg/mL + 1 mg/mL |
| | *B. longum* CBi0703+ Vitamin C (Vit C) | 200 µg/mL + 200 µg/mL |
| | *B. longum* CBi0703+ EPA plus (EPA) | 200 µg/mL + 200 µg/mL |
| | *B. longum* CBi0703+ Collagen (Col) | 200 µg/mL + 1 mg/mL |
| | *B. longum* CBi0703+ Chondroitin sulphate (CS) | 200 µg/mL + 1 mg/mL |
| | *B. longum* CBi0703+ Glucosamine sulphate (GS) | 200 µg/mL + 1 mg/mL |
| | *B. longum* CBi0703+ CS and GS mix (CM) | 200 µg/mL + 1 mg/mL |
| | *B. longum* CBi0703 + cat's claw | 200 µg/mL + 10 µg/mL |
| | *B. longum* CBi0703+ turmeric acid (TM) | 200 µg/mL +200 µg/mL |
| | *B. longum* CBi0703+ mangosteen extr | 200 µg/mL + 1 µg/mL |
| | *B. longum* CBi0703+3SL | 200 µg/mL + 200 µg/mL |
| | *B. longum* CBi0703 +6SL | 200 µg/mL + 500 µg/mL |
| | *B. longum* CBi0703+3 SL_ 6SL | 200 µg/mL + 200 µg/mL+ 500 µg/mL |
| | *B. longum* CBi0703 + calanus oil | 200 µg/mL + 200 µg/mL |
| | *B. longum* CBi0703+ CBD | 200 µg/mL + 10 µg/mL |
| | *B. longum* CBi0703 + resolvins | 200 µg/mL + 10 µg/mL |
| | *B. longum* CBi0703+ pimpinella anisum | 200 µg/mL + 50 µg/m |
| | *B. longum* CBi0703 + VFP46 | 200 µg/mL + 10⁷ CFU/mL |
| | *B. longum* CBi0703 + larazotide (LZT) | 200 µg/mL + 20 µg/mL |
| | *B. longum* CBi0703+ Egg-shell membrane (NEM) | 200 µg/mL + 100 µg/mL |

SW1353 cells (1 x 10⁶ cells/well) were seeded in 96-well plates for 24 h. After 24 h, cells were rinsed with PBS (phosphate buffered saline) and treated for 2 hours with H₂O₂ 50 µM. Then, cells were rinsed with PBS and treated for 22 hours with the combinations. In this line, H₂O₂ is the major source of endogenous reactive oxidative stress (ROS), and it has been extensively used to induce oxidative stress in *in vitro* models and to induce apoptosis in several types of cells, including the human chondrosarcoma-derived cell line SW1353 (Pang et al., 2021). To evaluate cell viability after H₂O₂ exposure and posterior 22-hour treatment neutral red assay was performed. Briefly, chondrocytes were exposed with 100 µL of neutral red (5 mg/mL). The plates were then incubated for 3 h with a medium containing neutral red in darkness at 37 °C. The cells were subsequently washed, the dye was extracted in each well with decolourant solution (ethanol, glacial acetic acid and water solution) for 10 minutes shacking and the absorbance was read using a spectrophotometer at an optical density of 540 nm. Cells treated with DMSO 50 % were used as negative control for cell viability. For ingredients *B. longum* CBi0703, Col, GS, CS and Vit C a cell viability study without H₂O₂ stimulation was initially performed to establish the experimental dose for these ingredients in the present study.

### Cell Proliferation assay

Cell proliferation was determined with the BrdU incorporation into the *de novo* DNA synthesized in proliferating cells (Flomerfelt & Gress, 2016) using a colorimetric ELISA assay following manufacturer instructions (Abcam, UK). Four replicates were analysed for each experimental condition evaluated. SW1353 cells (1 x 10⁶ cells/well) were seeded in 96-well plates for 24 h. After 24 hours, cells were rinsed with PBS and treated for 2 hours with H₂O₂ 50 µM. Then, cells were rinsed with PBS and treated for 22 hours with the combinations.

### Annexin V-FITC

To evaluate the effect of treatments on OA chondrocytes apoptosis, annexin V-FITC and Apostat were determined by flow cytometry. In viable eukaryotic cells, the negatively charged phosphatidylserine (PS) phospholipid is in the cytosolic side of the lipid bilayer of the cell membrane. Annexin V has a high affinity for PS. Apoptotic cells are positively stained due to a redistribution to the external side of PS during apoptosis but are negative for staining with propidium iodide (PI). This analysis was used for early or late apoptosis evaluation. In this case, Annexin V-FITC Kit by Milteny Biotec (Germany) was used, following manufacturers manual (140-001-403.05). SW1353 cells (1 x 10⁶ cells/well) were seeded in 12-well plates for 24 h. After 24 hours, cells were rinsed with PBS and treated for 2 hours with H₂O₂ 50 µM. Then, cells were rinsed with PBS and treated for 22 hours with the combinations. Apostat was used for caspases activity evaluation (Köhler C., Orrenius, S., & Zhivotovsky, B. (2002). Evaluation of caspase activity in apoptotic cells. Journal of Immunological Methods, 265(1-2), 97-110. https://doi.org/10.1016/s0022-1759(02)00073-x). This measurement was conducted using Intracellular Caspase Detection Apostat (Biotechnne).

### RT-qPCR

For the evaluation of the gene expression of different genes of interest, the Applied Biosystem High-Capacity cDNA Reverse Transcription Kit (4368814) and Roche LightCycler 480 SYBR Green I Master (04707516001) were used. The protocols described in both kits were followed. 3 replicates were analysed for each experimental condition evaluated. After treatments, total RNA was extracted from the SW-1353 cells using Biosystem High-Capacity cDNA Reverse Transcription Kit (4368814) and Roche LightCycler 480 SYBR Green I Master (04707516001) were used) according to the manufacturer's instructions. Total RNA was transcribed into cDNA by PrimeScript-RT reagent kit, then the cDNA was amplificated by the SYBR Premix Ex Taq (Sangon). The cycling conditions were 40 cycles at 50 °C for 2 min, 95 °C initial denaturation for 10 min, 95 °C denaturation for 15 seconds, and 60 °C annealing for 30 seconds. The mRNA encoding each target was measured using real-time PCR and *GAPDH* was used as housekeeping gene. The cycle threshold (Ct) value of the target gene was normalized to GAPDH. The primers and amplification products of each gene used in for the osteoarthritis model are shown in **Table 2.**

**Table 2. Primer sequences for the osteoarthritis model.**

| **Target Gene** | **Fw sequence** | **Rv sequence** |
|---|---|---|
| *COL1A1* | CTCCTCGCTTTCCTTCCTCT | AATGGTGCTCCTGGTATTGC |
| *COL2A1* | CAACACTGCCAACGTCCAGAT | CTGCTTCGTCCAGATAGGCAAT |
| *COL10A1* | GCTTCAGGGAGTGCCATCA | CGGCATTGGGAAGCTGG |
| *SOX9* | ACACACAGCTCACTCGACCTTG | GGAATTCTGGTTGGTCCTCTCTT |
| *AGC1* | ACTTCCGCTGGTCAGATGGA | TCTCGTGCCAGATCATCACC |
| *TIMP1* | AGATAGCCTGAATCCTGCC | CTGGGTGGTAACTCTTTATTTC |
| *ECM1* | GTAACAGCCACCAGACAAGC | TCTGTCCTGTAGCCGTGAAG |
| *MMP1* | CTGTTCAGGGACAGAATGTGCT | TCGATATGCTTCACAGTTCTAGGG |
| *MMP13* | TCCTCTTCTTGAGCTGGACTCATT | CGCTCTGCAAACTGGAGGTC |
| *GBL1* | ATACTGGCTGGCTAGATCACTG | GGCAAAATTGGTCCCACCTATAA |
| *Ki67* | GAAAGAGTGGCAACCTGCCTTC | GCACCAAGTTTTACTACATCTGCC |
| *GADPH* | CATGAGAAGTATGACAACAGCCT | AGTCCTTCCACGATACCAAAGT |

The primers used for the different genes were obtained from Biomers.net (Ulm, Germany). The fold changes in the mRNA levels were calculated as a percentage of the control group using the -2^{ΔΔCt} method (Livak, K. J., & Schmittgen, T. D. (2001). *Methods (San Diego, Calif.), 25*(*4*)*,* 402-408. https://doi.org/10.1006/meth.2001.1262 with *GADPH)* (glyceraldehyde-3-phosphate dehydrogenase) as an endogenous control.

### Statistical analyses

For each determination, an initial exploration was carried out to rule out discrepant points within the groups. For this purpose, the Grubbs statistical test was used, using the GraphPad Prism 9 software (GraphPad Software, Inc., La Jolla, CA, USA). All data are expressed as means ± standard error of the mean (SEM). To analyse differences in the different parameters, a t-student test to analyse differences of every treatment to a vehicle. A probability level of p<0.05 was defined as statistically significant and a probability below 0.1 was defined as trending toward significance.

### Results

### Evaluation of the properties of ingredient combinations on chondrocytes viability and proliferation

Although a reduction in viability with GS or the combination of *B. longum* and Vit C was statistically observed, it did not fall below 80%, the threshold considered biologically significant, because of the inherent variability of the technique **(****Figure 1A****).** Interestingly, combinations of *B. longum* CBi0703 with GS, CM and NEM, induced cell growth and proliferation in osteoarthritis conditions. In this line, in OA conditions, human chondrocytes treated with Col, *B*. *longum* CBi0703 and combinations of *B. longum CBi0703* with Col, CS, CM and NEM, prevented the cartilage degradation as observed in chondrocyte proliferation **(****Figure 1B****).**

### Evaluation of the properties of ingredient combinations on chondrocytes apoptosis

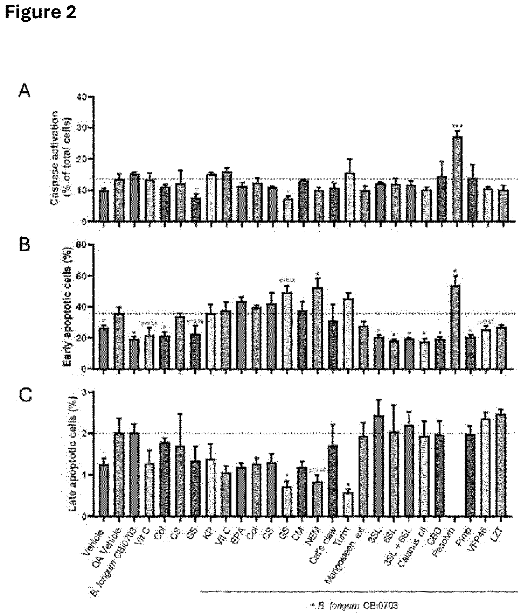

In OA, chondrocytes undergo apoptosis due to factors like mechanical stress, inflammation, and oxidative stress. This programmed cell death reduces cartilage integrity and function, contributing to joint degeneration and pain. Apoptosis in chondrocytes accelerates cartilage breakdown, exacerbating the progression of osteoarthritis. As pointed for the other models, caspase activation develops a critical role in the chondrocyte's apoptosis. GS and combination of GS with *B. longum* CBi0703 reduced the caspase activation typically observed in osteoarthrosis **(****Figure 2A****).** It is well-established that OA induces apoptosis in chondrocytes leading to an increase in early apoptotic cells. Single treatment with *B. longum* CBi0703 or Col, reversed this process, and Vit C or GS tended to revert this fact **(****Figure 2B****).**

The induction of OA increased the late apoptotic cells **(****Figure 2C****),** whereas the combinations of *B. longum* CBi0703 with GS and NEM, reverted this process.

### Evaluation of B. longum CBi0703 and combinations on osteogenesis

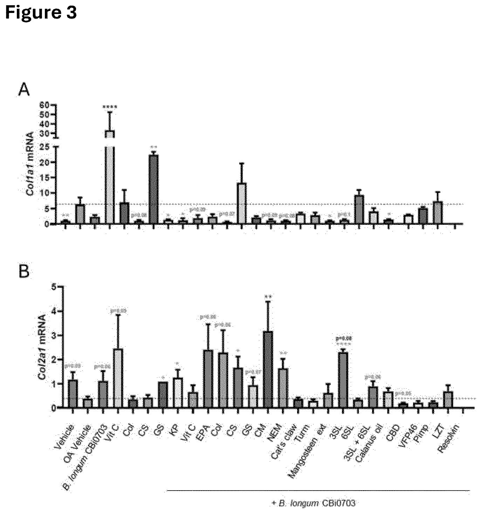

COL1A1, a fibroblastic marker expressed in dedifferentiated chondrocytes, shows elevated expression in OA. This upregulation was particularly pronounced in OA chondrocytes treated with Vit C or GS **(****Figure 3A****).** Contrarily, the combinations of *B. longum* CBi0703 with Vit C reverted this process and tended to reverse in the combinations with CS and NEM.

COL2A1 is crucial for maintaining cartilage integrity. In OA, decreased *COL2A1* expression leads to weakened cartilage structure, accelerating degeneration and joint damage. In the present study, *COL2A1* expression was reduced in OA **(****Figure 3B****).** On the other hand, chondrocyte treatment with the combinations of *B. longum* CBi0703 with other compounds, specially, CS, CM and NEM, reverted this process.

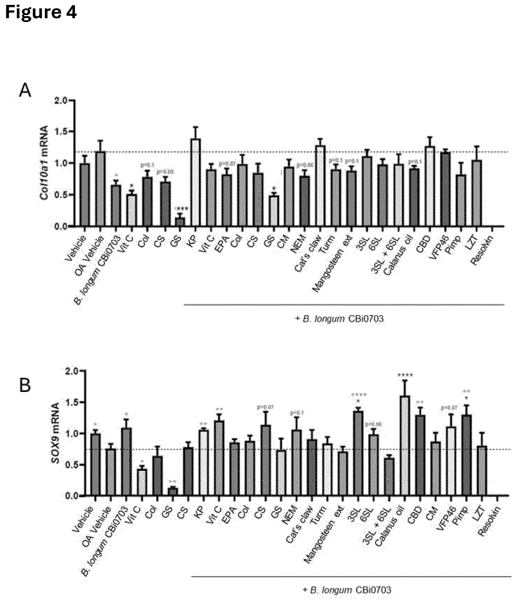

*COL10A1* expression in chondrocytes is associated with OA as it indicates hypertrophic differentiation, contributing to cartilage calcification and degradation, thus worsening the disease progression. *COL10A1* expression was slightly increased in OA **(****Figure 4A****),** whereas treatment with GS or the combinations of *B. longum* CBi0703 with GS, downregulated *COL10A1* expression.

Finally, SOX9 plays a crucial role in chondrocyte function by regulating cartilage matrix synthesis. Its reduced expression in OA leads to impaired cartilage repair and accelerated degeneration. *SOX9* expression was reduced in OA, especially in chondrocytes treated with Vit C or GS **(****Figure 4B****).** In contrast, chondrocytes treatment, in an OA environment, with *B. longum* CBi0703 individually or in combination with Vit C, prevented this process. In addition, *B. longum* CBi0703 with CS tended to increase *SOX9* expression.

### Evaluation of B. longum CBi0703 and combinations on articular degradation

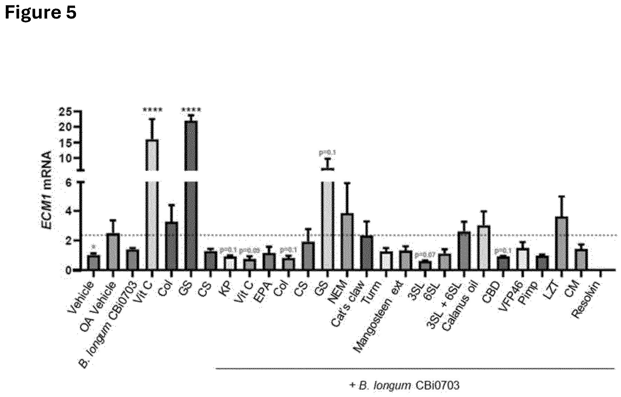

*ECM1* expression in chondrocytes contributes to articular degradation by disrupting extracellular matrix organization, promoting cartilage breakdown, and exacerbating osteoarthritis progression. *ECM1* expression was determined in human chondrocytes exposed to an osteoarthritic environment, observing an increased expression in OA, especially in cells treated with Vit C or GS **(****Figure 5****),** whereas treatment with the combinations of *B. longum* CBi0703 with Vit C tended to prevent this process.

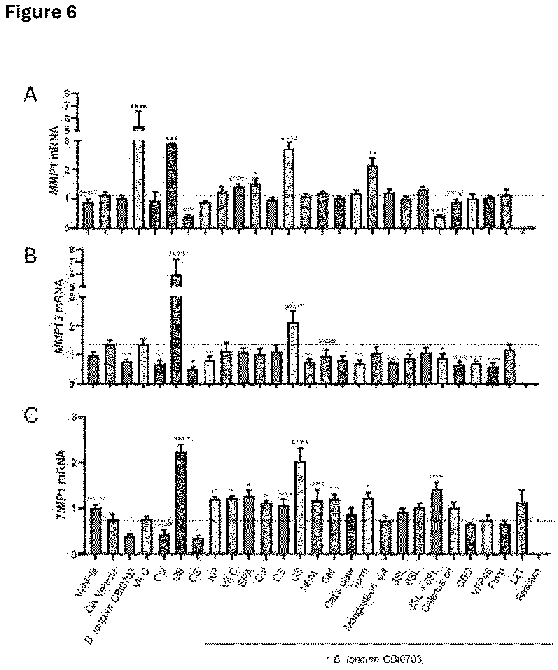

MMP1 plays a pivotal role in the degradation of articular cartilage. It facilitates the breakdown of collagen fibres within the extracellular matrix, compromising the structural integrity of cartilage. This process contributes to the progressive erosion of joint surfaces observed in OA, highlighting MMP1's significant involvement in the pathogenesis of this degenerative disease. *MMP1* expression was analysed observing a slightly induction in OA and strongly in chondrocytes treated with Vit C and GS, or *B. longum* CBi0703 combined with Col or GS **(****Figure 6A****).** In contrast, CS treatment decreased *MMP1* expression.

MMP13 is a metalloproteinase whose expression in chondrocytes speeds up joint degradation by degrading collagen in the extracellular matrix, causing cartilage breakdown and worsening OA. *MMP13* expression was analysed in human chondrocytes exposed to osteoarthritic environment, observing an up-regulation in the *MMP13* expression in OA and specially after the GS treatment **(****Figure 6B****).** In contrast, treatment with Col, CS and *B. longum* CBi0703 individually and in combination with NEM, reverted this process.

In chondrocytes, TIMP1 regulates the degradation of articular cartilage by inhibiting MMPs (matrix metalloproteinases), including MMP1 and MMP13. This process helps to maintain the balance between cartilage matrix breakdown and repair, crucial in OA progression, and consequently, higher levels of TIMP1 protect from articular degradation. *TIMP1* expression was downregulated in OA as well in chondrocytes treated with *B. longum* CBi0703, Col and CS **(Figure 6C).** Surprisingly, treatment with GS and *B. longum* CBi0703 in combination with Vit C, Col, GS and CM prevented this process, upregulating the *TIMP1* expression, supporting that these combinations are useful in the treatment of OA in a subject having OA or as a prophylactic treatment.

### Evaluation of B. longum CBi0703 on cartilage integrity and articular senescence

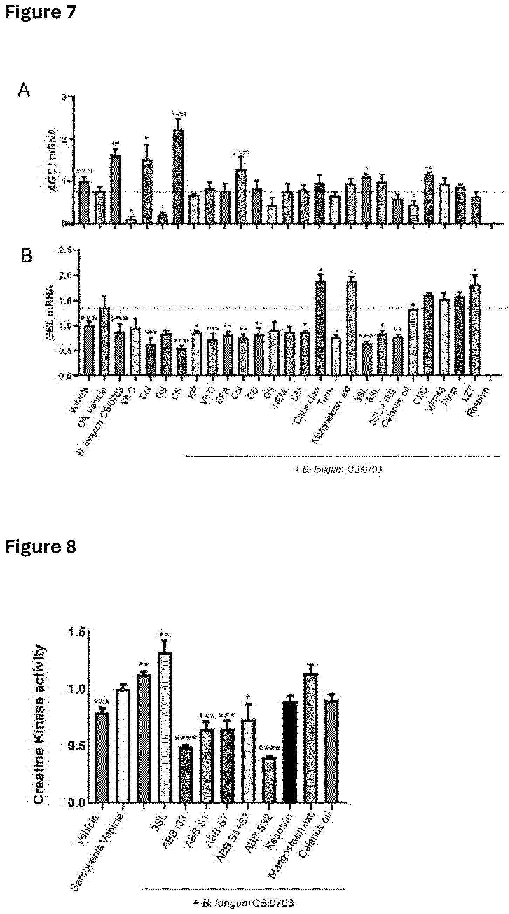

Regarding the cartilage integrity, AGC1 (also known as Aggrecan) expression in chondrocytes is crucial for maintaining cartilage integrity by promoting the assembly of proteoglycans in the extracellular matrix. This helps resist compression and supports joint function in OA. *AGC1* expression was slightly reduced in OA and specially in chondrocytes treated with Vit C or GS **(****Figure 7A****).** On the other hand, chondrocytes treated with Col, CS and *B. longum* CBi0703, reverted this process.

GBL (beta-galactosidase) is implicated in joint senescence by accumulating senescence-associated beta-galactosidase (SA-β-gal). In our model, *GBL* expression was increased in OA **(****Figure 7B****).** Contrary, several of the tested treatments reverted this process, highlighting Col, CS and *B. longum* CBi0703 alone or in combination with Vit C, Col, CS and CM. These results suggest a promising benefitial effect of several of ingredients and combinations studied.

### Discussion

The results shows benefits of a combination of a *B. longum* biotic (lysate) and further joint-supportive bioactives, which may be found in egg-shell membrane, notably collagen, glucosamine, chondroitin for use in an innovative therapeutic approach for OA.

By focusing on the prevention of cartilage catabolism, a approach is offered compared to current available options, which predominantly target anabolic processes. Current nutraceutical approaches to manage OA primarily focus on enhancing cartilage anabolism-stimulating the production of extracellular matrix components to rebuild damaged cartilage (Chen, T., Weng, W., Liu, Y, Aspera-Werz, R. H., Nüssler, A. K., & Xu, J. (2021). Update on Novel Non-Operative Treatment for Osteoarthritis: Current Status and Future Trends. Frontiers in Pharmacology, 12. https://doi.org/10.3389/fphar.2021.755230).

While these treatments can slow the progression of joint damage, their efficacy is often limited by the persistent imbalance between anabolic and catabolic processes, favoring degradation over repair. In contrast, the mechanism of action observed for combinations according to the invention in this study emphasizes a reduction in cartilage catabolism, effectively avoiding or slowing the degradation of articular tissues. By downregulating matrix metalloproteinases and aggrecanases, combinations according to the invention mitigate the breakdown of the extracellular matrix, preserving cartilage integrity. This distinction highlights the complementary potential of combinations according to the invention in addressing the multifactorial nature of OA pathophysiology. In particular, the results support a synergistic effect of a combination of the *B. longum* post biotic (lysate) and one or more further joint-supportive bioactive compounds, such as glucosamine, chondroitin, collagen or egg shell membrane (comprising such bioactive compounds), the combination with another bioactive ingredient, such as peptides from kefir-fermented milk or HMO (·SL, 6SL or both) and the combination with omega-3 derivatives and SPMs (such as resolvin) or botanical extracts such as CBD. Herewith the present invention provides a more comprehensive approach to OA management. In addition, an enhanced chondrocyte viability is obtained, protecting chondrocytes from apoptosis and senescence ensuring the maintenance of functional cartilage cells as well as encouraging the growth of chondrocytes to support the repair and regeneration of damaged cartilage. In addition, some combinations, such as, *B. longum* CBi0703 biotic with CS or *B. longum* CBi0703 post-biotic with GS, showed synergistic effects compared to individual treatments. Particularly, CS and *B. longum* CBi0703 post-biotic together significantly enhanced *Col2a1* expression, and GS and *B. longum* CBi0703 post-biotic together significantly reduced late apoptotic cells compared to the individual treatments. This suggests that the postbiotic may boost the chondroprotective action of established OA supplements. The observed synergy with chondroitin sulphate and glucosamine sulphate indicates potential for combinatorial therapies to more effectively address OA pathophysiology than single interventions.

It is considered that the integration of the combination according to the invention into clinical practice as a complementary approach to existing OA treatments enhances therapeutic outcomes. By addressing catabolic processes, *B. longum* post-biotic (such as CBi0703 post-biotic) this postbiotic can be used to complement the anabolic effects of disease-modifying OA drugs (DMOADs), such as glucosamine and chondroitin sulfate. This combination may against other be used for one or more of the following: to protect against joint degradation, enhance symptom relief by simultaneously reducing inflammation, promoting cartilage repair, decrease the required dosages of other pharmacological agents, potentially minimizing side effects associated with long-term use.

As a postbiotic, *B. longum* (such as CBi0703) combined with the DMAOD may also be used to address systemic low-grade inflammation through gut-joint axis modulation, contributing to overall joint health.

### Example 4 Osteoarthritis (B.longum plus prebiotics)

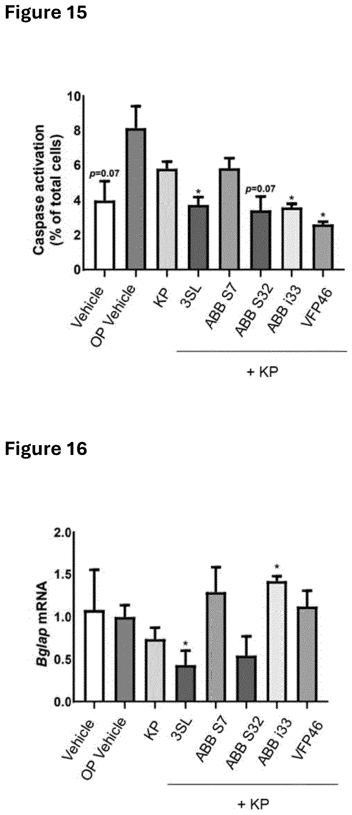

Cell viability was tested for various biotics and combinations thereof, using an osteoarthtitic model (Ref: Pang KL, Chow YY, Leong LM, Law JX, Ghafar NA, Soelaiman IN, et al. Establishing sw1353 chondrocytes as a cellular model of chondrolysis. Life 2021;11:1-17. https://doi.org/10.3390/life11040272). However, a combination of Bidobacterium lysate (*B longum,* strain Cbi0703) and sillyllactose (3SL+6SL) induced cell growth and proliferation (cell viability) in osteoarthritis conditions. The effect of Bifidobacterium lysate+3SL+6SL was synergistically superior to Bifidobacterium lysate+3SL alone and Bifidobacterium lysate+6SL alone. Several other combinations with Bifidobacterium lysate also had a positive effect, e.g. with egg shell membrane (NEM) Results are shown in Figure 15.

### Chondrocytes proliferation

In OA conditions, chondrocyte proliferation counteracts cartilage degradation. The *B. longum* postbiotic with 3SL or 6SL increased the chondrocyte proliferation. Results are shown in Figure 16.

Ki67 can be used to assess proliferation of cells. Ki67 expression was reduced in OA, whereas the combination of *B. longum* with 3SL,+ optionally 6SL reverted this process, supporting that these treatments promote the proliferation of human chondrocytes in an osteoarthritic environment.

### Chondrocytes apoptosis

Osteoarthritis increased the early apoptotic cells, whereas combinations of *B.longum* lysate with 3SL+6SL reversed this process.

### Articular degradation

TIMP1 regulates the degradation of articular cartilage by inhibiting MMPs (matrix metalloproteinases). This maintains the balance between cartilage matrix breakdown and repair, and consequently, higher TIMP1 protects from articular degradation. Accordingly, TIMP1 expression was downregulated in the induced osteoarthritic model. Surprisingly, *B longum,* lysate with 3SL+6SL reverted this in a synergistic way, upregulating the TIMP1 expression.

### Articular senescence

GLB (beta-galactosidase) indicates increased cellular senescence. GLB was increased in OA. *B longum* lysate with 3SL, + optionally 6SL reverted this process. These results support a promising beneficial effect in osteoarthritis.

### Example 2: sarcopenia treatment

### Materials and method

An L6 myoblast model was used, as described in Rupert JE, Jengelley DHA, Zimmers TA. In Vitro, In Vivo, and In Silico Methods for Assessment of Muscle Size and Muscle Growth Regulation. Shock 2020;53:605-15. https://doi.org/10.1097/SHK.0000000000001498.

. The induction of the sarcopenia model was done using H₂O₂ (50 µM)

*B. longum* post-biotic was used, namely inactivated *B. longum CBi0703.* This post-biotic (200 ug/ml) was combined with various other putative actives:
Mangosteen extract (1 ug/ml) is a combination of xanthones derived from mangosteen extracts.
3SL is 3'-syalyllactose (200 ug/ml).
ABBS1 is *Saccharomyces boulardii* biotic added in an amount corresponding to 10⁸ cfu/ml.
ABBS7 is *Kluyveromyces marxianus* biotic added in an amount corresponding to 10⁸ cfu/ml.
Calanus oil is an oil rich in omega-3 fatty acids, added in an amount corresponding to 0.5 % (v/v).
Specialized pro-resolving mediators (SPM) including Resolvin is a known specialized pro-resolving mediator derived from omega-3 fatty acids, added in an amount corresponding to 0.5 % (v/v)..
ABB S32 is *Saccharomyces cerevisiae* biotic, and was added in an amount corresponding to 10⁶ cfu/ml.
ABB i33 is a yeast extract (Torula), added in an amount of 200 ug/ml.

### Results

### Muscle damage

Sarcopenic conditions increased the creatine kinase activity compared to the physiological conditions (control vehicle). Interestingly, the combination of *B*. *longum* post-biotic + yeast extract (Torula) reduced creatine kinase activity compared to *B. longum* alone as well as to the sarcopenic stimulus. This suggests a of this combination in the treatment of muscular sarcopenia, attenuating the muscle damage. Results are shown in Figure 8.

### Muscle senescence

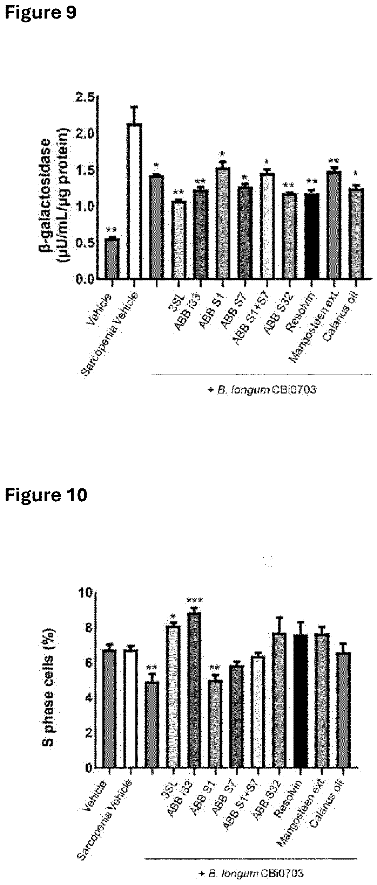

The beta-galactosidase enzyme activity is indicative of senescent cells, contributing to muscle dysfunction and loss. Remarkably, a reduction of beta-galactosidase activity was observed after the treatment with all combinations (approximately 50% compared with the sarcopenic condition), which supports a beneficial effect of all tested treatments in the cellular senescence in sarcopenia. Results as shown in Figure 9.

### Muscle cell proliferation

A reduction in the muscular cell proliferation it typical in sarcopenia. In sarcopenic conditions, a significant drop in the S phase cells in myoblasts treated with a single biotic (*B.longum* post-biotic) was observed. In contrast, a combination of *B. longum* post biotic plus yeast extract (TorulaABB i33) or SPM rich in resolvins increased the S phase cells, supporting the effectivity in increasing cell proliferation. Results are shown in Figure 10.

### Muscle apoptosis

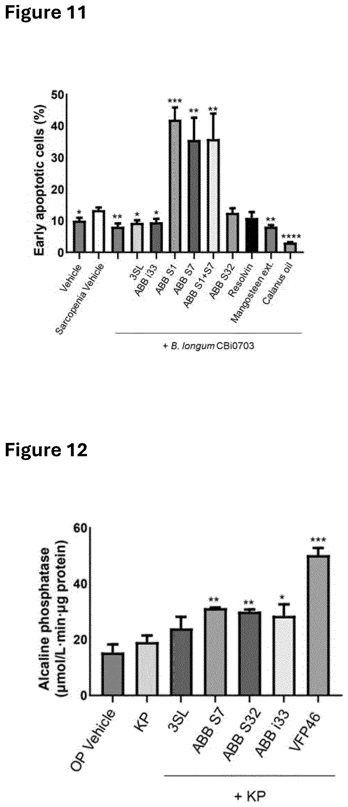

As expected, early apoptotic cells increased in the sarcopenic conditions. The combination of *B longum* post biotic + yeast extract (Torula) reduced apoptotic cells, supporting that a combination according to the invention is suitable to protect myoblasts from apoptosis in sarcopenia. Results are shown in Figure 11.

### Summary

Cell senescence (= muscle dysfunction and loss) was reduced with all tested combinations. A combination of *B. longum* postbiotic plus yeast extract (Torula) or resolvins presented beneficial effects in the highest number of determinations, including beneficial effects in relation to muscle damage, apoptosis and cell proliferation.

### Example 3 osteoporosis treatment

MC3T3-E1 osteoblasts were used in the osteoporosis model as described in Song Y, Ju Y, Song G, Morita Y. In vitro proliferation and osteogenic differentiation of mesenchymal stem cells on nanoporous alumina. Int J Nanomedicine 2013;8:2745-56. https://doi.org/10.2147/IJN.S44885). The induction of the osteoporosis model was done using H₂O₂ (400 µM) during 24 h in combination with the treatment.

As a first biotic a fermentation product, containing peptides was used (kefir fermented milk powder; Kefpep^{®}, obtained from PhermPep^{®}, Taiwan) providing at least 8 mg/g peptides. The fermentation product (1 mg/l) was used in combination with one of the following:
3SL: 3'-syalyllactose) in an amount of 1 mg/ml.
*Kluyveromyces marxianus* (ABB S7 in an amount of 10⁸ cfu/ml
*Saccharomyces cerevisiae* (ABB S32) in an amount of 10⁶ cfu/ml
Yeast extract from Torula (Provesta) in an amont of 1 mg/ml
VFP24: *Lactiplantibacillus plantarum* in an amount of 10⁷ cfu/ml

### Results

### Bone damage

Alkaline phosphatase activity assesses large changes in bone turnover. Indeed, low levels of this marker are associated with bone damage. Peptidic postbiotic (Kefpep)+ at least one of postbiotics S7 , yeast extract (Provesta), or probiotics *Saccharomyces cerevisiae* ABB S32, *Lactiplantibacillus plantarum* VFP24 increased alkaline phosphatase, supporting a protective effect of these combinations in bone damage associated with osteoporosis. Results are shown in Figure 12.

### Bone senescence

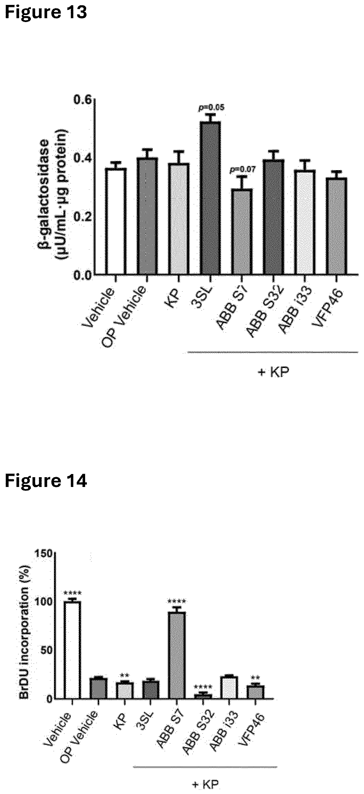

Cellular senescence in osteoblasts contributes to bone aging. beta-galactosidase increased in osteoporotic conditions, but decreased after **Kefpep + S7,** which supports protection from senescence associated to bone osteoporosis. Results are shown in Figure 13.

### Osteoblast proliferation

Reduced osteoblast proliferation impairs bone formation and contributes to bone degeneration. Using the BrDU assay, the osteoporotic condition reduces cell proliferation. Interestingly, cell proliferation was recovered after Kefpep + S7, supporting a specific advantage for the combination according to the invention. Results are shown in Figure 14.

### Bone apoptosis

Osteoarthritis rises caspase activation. Peptidic postbiotic (Kefpep) + yeast extract (Provesta) reduced this caspase activation, supporting protection from osteoblast apoptosis induced in osteoarthritis. Results are shown in Figure 15.

### Bone formation

In osteoporotic conditions, peptidic postbiotic plus yeast extract (Kefpep + Provesta) increased the expression of Bglap, related with bone remodelling, supporting a positive effect in promoting bone formation. Results are shown in Figure 16.

### Summary

All tested combinations exhibit beneficial effects related to bone osteoporosis. Peptidic biotic (KefPep) as such had negative effects in cell proliferation. To recover this, the combination with ABB S7 was particularly useful. This combination presented particularly beneficial effects on viability, bone turnover, senescence, apoptosis, osteoblast proliferation and bone resorption. The combination of peptidic biotic plus yeast extract (Kefpep + ABB i33), displayed positive effects on bone turnover, downregulation of caspase activation, and bone formation, and did not present negative effects in any of the evaluated aspects.

## Claims

1. A combination of at least a first bioactive selected from the group consisting of probiotics, parabiotics, postbiotics and prebiotics, and at least a second bioactive selected from the group consisting of probiotics, parabiotics, postbiotics, prebiotics, botanical extracts, omega-3 polyunsaturated fatty acids and further joint-supportive substances, wherein said first bioactive and said second bioactive are different from each other, for use in the treatment of a medical musculoskeletal degenerative disorder.

2. A combination for use according to claim 1, wherein the medical disorder is sarcopenia.

3. A combination for use according to claim 1 or 2, wherein said first bioactive is *a Bifidobacterium* lysate, and said second bioactive is a yeast extract, the yeast extract preferably comprising nucleotides and vitamins B.

4. A combination for use according to claim 3, wherein said first bioactive is *a Bifidum longum* lysate, and said second bioactive is a yeast extract, the yeast extract comprising nucleotides and vitamins B, preferably two or more vitamins B selected from the group of B1 (thiamine), B2 (riboflavin), B3 (niacin), B5 (pantothenic acid), B6 (pyridoxine), B7 (biotin), B9 (folic acid), and B12 (cobalamin).

5. A combination for use according to any of the preceding claims, wherein said second bioactive is a Torula extract.

6. A combination for use according to claim 1, wherein the medical disorder is osteoporosis or osteopenia.

7. A combination for use according to claim 1 or 2, wherein said first bioactive is a yeast extract and said second bioactive is selected from kefir-peptides, kefir-fermented milk powder, bioactive is a peptidic post-biotic, *Lactiplantibacillus plantarum* and *Saccharomyces cerevisiae.*

8. A combination for use according to claim 7, wherein the the yeast extract is a Torula yeast extract.

9. A combination for use according to claim 7 or 8, comprising a a viable yeast, which may be said *Saccharomyces cerevisiae* or a further viable yeast.

10. A combination for use according to claim 9, wherein the further viable yeast is *K. marxianus.*

11. A combination for use according to claim 1, wherein the medical disorder is osteoarthritis.

12. A combination for use according to claim 11, wherein said first bioactive is a *B. longum* lysate, said second bioactive is sillyllactose 3SL and the combination further comprises a third bioactive, which third bioactive is sillyllactose 6SL.

13. A combination for use according to claim 11 or 12, wherein said first bioactive is a *B. longum* biotic and said second bioactive comprises a joint-supportive compound selected from the group of chondroitins, glucosamins, collagens, hyaluronic acids, dermatans, keratans, desmosines, and elastins.

14. A combination for use according to claim 13, wherein the *B. longum* biotic is a *B. longum* lysate and wherein the combination comprises the joint-supportive compounds glucosamine and chondroitin.

15. A combination for use according to any of the preceding claims, wherein the combination comprises a *B. longum* CBi0703 biotic or a *B. longum* CBi0703 derivative strain biotic, preferably a *B. longum* CBi0703 postbiotic or a *B. longum* CBi0703 derivative strain postbiotic, in particular a *B. longum* CBi0703 lysate or a *B. longum* CBi0703 derivative strain lysate.

16. A pharmaceutical or nutraceutical composition comprising at least a first bioactive and at least a second bioactive, wherein said first biotic and said second biotic are as defined in any of the preceding claims.
